# EUROPEAN PATENT APPLICATION

(11) **EP 3 338 771 A1**
(43) Date of publication of application: **27.06.2018**
(21) Application number: 17798843.3
(22) Date of filing: 11.05.2017
(51) Int. Cl.: A61K 31/135, A61K 31/444, A61P 25/04, A61P 29/00

(54) **PHARMACEUTICAL COMPOSITION OF A COMBINATION OF TRAMADOL-ETORICOXIB HYDROCHLORIDE FOR THE TREATMENT OF PAIN**

(30) Priority: 18.05.2016 MX 2016006464
(71) Applicant: Laboratorios Liomont, S.A. de C.V., 05000 Distrito Federal (MX)
(72) Inventor: SALTO RIVERA, Reyna Astrid, Ciudad de México 05000 (MX); WALLS FLORES, Oliver Daniel, Ciudad de México 05000 (MX); RODRÍGUEZ HERNÁNDEZ, Ramón, Ciudad de México 05000 (MX)
(74) Representative: Padial Martinez, Ana Belen
(86) International application number: PCT/IB2017/052775
(87) International publication number: WO 2017/199140

(57) **Abstract**

The invention relates to a method and a pharmaceutical composition of a combination of tramadol-etoricoxib hydrochloride for the treatment of pain, the production method of which is based on two active ingredients consisting of granules and/or multi-particulates with tramadol hydrochloride and granules and/or multi-particulates with etoricoxib, which can be contained in a mixture of powders, or included in a two-layer tablet with or without a coating or in a capsule as granulates and/or tablets with or without a coating.

## Description

### FIELD OF THE INVENTION

The present invention consists in an orally administered pharmaceutical composition of a drug containing the tramadol-etoricoxib hydrochloride for the treatment of pain.

### BACKGROUND OF THE INVENTION

The active ingredient etoricoxib, is marketed in Mexico as the reference product and with the trade name. Etoricoxib is a highly selective cyclooxygenase-2 (COX-2) inhibitor. The chemical name of etoricoxib is 5-chloro-2-(6-methylpyridin-3-yl)-3-(4-methylsulfonylphenyl)pyridine with an empirical formula C₁₈H₁₅CIN₂O₂S, whose therapeutic indications are for the acute and chronic treatment of the signs and symptoms of osteoarthritis and rheumatoid arthritis, ankylosing spondylitis, acute gouty arthritis, to relieve acute and chronic pain, primary dysmenorrhea, moderate to severe acute post-surgery pain associated with dental surgery and moderate to severe acute post-surgery pain associated with gynecological abdominal surgery.

The active ingredient tramadol hydrochloride has dual activity as it has a great agonistic activity on central opioid receptors as well as it inhibits the re-uptake of norepinephrine and serotonin in the central nervous system, preventing the transmission of pain throughout the bone marrow. The chemical name of tramadol hydrochloride is (1R,2R)-2-(dimethylaminomethyl)-1-(3-methoxyphenyl)cyclohexan-1-ol hydrochloride with an empirical formula C₁₆H₂₆CINO₂, whose therapeutic indications are for the treatment of moderate pain, intense moderate and neuropathic pain, in the adjuvant treatment of osteoarthritis and in the treatment of restless legs syndrome and post-surgery tremors.

From the search of patents in the drugs gazette of the selective cyclooxygenase-2 inhibitor Etoricoxib, it was found that patent MX 208595 covers its use as active ingredient until July 8, 2017. From the search in IMPI's database, it was found that patent MX313106 covers the use of intermediate product for the synthesis of etoricoxib; it was found that patent application MX/a/2017/005316 covers the manufacturing of a granulate by fluid bed granulation. From the international search, we found that patent WO 2014/033526 covers the manufacture of tablets by dry-path to retain the polymorph shape of etoricoxib. In this search, we found references covering the topical pharmaceutical form of etoricoxib, of the synthesis and use thereof. Patent WO 2004/093811 covers the combination of use of a sodium channels blocker with selective cyclooxygenase-2 inhibitors. Patent WO 2004/093813 covers the combination of use of a calcium blocker modulator with selective cyclooxygenase-2 inhibitors.

The patent of use as active ingredient of the tramadol hydrochloride is of the public domain. In reviewing IMPI's drugs gazette we found that patent MX266401 covers the use of the combination of ketorolac with tramadol hydrochloride for the treatment of pain in an oral administration, that patent MX230075 covers the use of the combination of tramadol hydrochloride and the selection of an anticonvulsant, patent MX275811 which covers the use of the combination of tramadol hydrochloride with lysine clonixinate to have an analgesic pharmaceutical composition.

These revisions did not show any combination that covers selective cyclooxygenase-2-inhibitors as is the special case of etorixocib with tramadol hydrochloride for the treatment of pain.

Based on the above, there is the accuracy that this combination of etoricoxib with tramadol hydrochloride for oral administration is a pharmaceutical product having a high degree of technological innovation as compared to the described backgrounds, so it qualifies as a beneficial product for Mexican and international societies for the treatment of pain-related allopathy.

### OBJECT OF THE INVENTION

It consists in providing a pharmaceutical composition as a new alternative for the treatment of pain.

A further object of the invention is to have an alternative of dosing in a single intake the two active ingredients etoricoxib-tramadol hydrochloride.

Another object of the invention is to provide a combination of etoricoxib-tramadol hydrochloride in a pharmaceutical form that may by orally administered.

### DETAILED DESCRIPTION OF THE INVENTION

For such reasons, a novel pharmaceutical composition of the combination of etoricoxib with tramadol hydrochloride to be orally administered has been developed, which was found to have unexpected effects, as well as the proposed concentration, which supersedes the effects existing until now; next, the process of manufacturing is described, which is not limitative but only to exemplify the processes:
1) The surfactant is dispersed in a solvent in a vessel.
2) With a mesh, sieve the etoricoxib or similar salts, one or more diluents, one or more binding agents and one or more disintegrants, add each component to a mixer and mix the powders.
3) With the dissolution of step 1, granulate the mixture of powders of step 2.
4) With a mesh, pass the granulate of step 3 and oven-dry.
5) With a mesh, pass the dry granulate of step 4 and mix with one or more lubricants and/or glidants and/or antiadherents.
6) In a tableting machine, compress the mixture obtained from step 4 to the required concentration between 10 mg and 120 mg of etoricoxib and/or similar salts.
7) In a vessel, disperse at least one tablet coating material in a solvent.
8) In a coating equipment, coat the tablets of step 6 with the dispersion of step 7.
9) In a vessel, disperse at least one binding agent in a solvent.
10) With a mesh, sieve the tramadol hydrochloride or similar salts, one or more diluents, add each component to a mixer and mix the powders.
11) With the dissolution of step 9, granulate the mixture of powders of step 10.
12) With a mesh, pass the granulate of step 11 and oven-dry.
13) With a mesh, pass the dry granulate of step 12 and mix with one or more lubricants and/or glidants and/or antiadherents.
14) In a tableting machine, compress the mixture obtained from step 13 to the required concentration between 10 mg and 30 mg of tramadol hydrochloride and/or similar salts.
15) In a vessel, disperse at least one tablet coating material in a solvent.
16) In a coating equipment, coat the tablets of step 14 with the dispersion of step 15.
17) Fill in capsules with the tablets obtained in steps 8 and 16 to the required dose of etoricoxib or similar salts and tramadol and/or similar salts.

### Example 1

1) The surfactant is dispersed in a solvent in a vessel.
2) With a mesh, sieve the etoricoxib or similar salts, one or more diluents, one or more binding agents and one or more disintegrants, add each component to a mixer and mix the powders.
3) With the dissolution of step 1, granulate the mixture of powders of step 2.
4) With a mesh, pass the granulate of step 3 and oven-dry.
5) With a mesh, pass the dry granulate of step 4 and mix with one or more lubricants and/or glidants and/or antiadherents.
6) In a vessel, disperse at least one solvent in a binding agent.
7) With a mesh, sieve the tramadol hydrochloride or similar salts, one or more diluents, add each component to a mixer and mix the powders.
8) With the dissolution of step 6, granulate the mixture of powders of step 7.
9) With a mesh, pass the granulate of step 8 and oven-dry.
10) With a mesh, pass the dry granulate of step 9 and mix with one or more lubricants and/or glidants and/or antiadherents.
11) In a tableting machine, compress the mixture obtained from steps 5 and 10 to the required concentration between 10 mg and 120 mg of etoricoxib and/or similar salts and between 10 mg and 300 mg of tramadol hydrochloride and/or similar salts.

### Example 2

1) The surfactant is dispersed in a solvent in a vessel.
2) With a mesh, sieve the etoricoxib or similar salts, one or more diluents, one or more binding agents and one or more disintegrants, add each component to a mixer and mix the powders.
3) With the dissolution of step 1, granulate the mixture of powders of step 2.
4) With a mesh, pass the granulate of step 3 and oven-dry.
5) With a mesh, pass the dry granulate of step 4 and mix with one or more lubricants and/or glidants and/or antiadherents.
6) In a vessel, disperse at least one binding agent in a solvent.
7) With a mesh, sieve the tramadol hydrochloride or similar salts, one or more diluents, add each component to a mixer and mix the powders.
8) With the dissolution of step 6, granulate the mixture of powders of step 7.
9) With a mesh, pass the granulate of step 8 and oven-dry.
10) With a mesh, pass the dry granulate of step 9 and mix with one or more lubricants and/or glidants and/or antiadherents.
11) In a tableting machine, compress the mixture obtained from step 10 to the required concentration between 10 mg and 300 mg of tramadol hydrochloride and/or similar salts.
12) Fill in capsules with the mixture of powders obtained in step 5 and with the tablets obtained in step 11 to the required dose of 10 mg to 120 mg of etoricoxib and/or similar salts and from 10 mg to 300 mg of tramadol hydrochloride and/or similar salts.

### Example 3

1) The surfactant is dispersed in a solvent in a vessel.
2) With a mesh, sieve the etoricoxib or similar salts, one or more diluents, one or more binding agents and one or more disintegrants, add each component to a mixer and mix the powders.
3) With the dissolution of step 1, granulate the mixture of powders of step 2.
4) With a mesh, pass the granulate of step 3 and oven-dry.
5) With a mesh, pass the dry granulate of step 4 and mix with one or more lubricants and/or glidants and/or antiadherents.
6) In a vessel, disperse at least one binding agent in a solvent.
7) With a mesh, sieve the tramadol hydrochloride or similar salts, one or more diluents, add each component to a mixer and mix the powders.
8) With the dissolution of step 6, granulate the mixture of powders of step 7.
9) With a mesh, pass the granulate of step 8 and oven-dry.
10) With a mesh, pass the dry granulate of step 9 and mix with one or more lubricants and/or glidants and/or antiadherents.
11) Fill in capsules with the mixture of powders obtained in step 5 and step 10 to the required dose of 10 mg to 120 mg of etoricoxib and/or similar salts and from 10 mg to 300 mg of tramadol and/or similar salts.

## Claims

1. A process and pharmaceutical composition of a combination of tramadol hydrochloride and/or similar salts with etoricoxib and/or similar salts for the treatment of pain, for oral administration.

2. The process and pharmaceutical composition of a combination of tramadol hydrochloride and/or similar salts with etoricoxib and/or similar salts according to claim 1, wherein the particle size of etorixocib and/or similar salts must be lower than or equal to a d90 lower than 30 microns.

3. The process and pharmaceutical composition of a combination of tramadol hydrochloride and/or similar salts with etoricoxib and/or similar salts according to claims 1 and 2, which cover granules or particulate material containing etoricoxib or similar salts and/or tramadol hydrochloride and/or similar salts individually or combined which allows for the oral co-administration thereof.

4. The process and pharmaceutical composition of a combination of tramadol hydrochloride and/or similar salts with etoricoxib and/or similar salts according to claims 1 to 3, which cover the oral administration in a concentration of 10 to 120 mg of etocoxib and/or similar salts with tramadol hydrochloride and/or similar salts in a concentration of 10 to 300 mg.

5. The process and pharmaceutical composition of a combination of tramadol hydrochloride and/or similar salts with etoricoxib and/or similar salts according to claims 1 to 4, which cover the oral administration in a concentration of 10 to 120 mg of etocoxib and/or similar salts with tramadol hydrochloride and/or similar salts in a concentration of 10 to 300 mg for the treatment of pain.

6. The use of a pharmaceutical composition of etoricoxib and/or similar salts in combination with tramadol hydrochloride and/or similar salts that may be administered individually or separately, at the same time or interchangeably one before the other, to suppress the effects of the pain.
